# EUROPEAN PATENT APPLICATION

(11) **EP 3 000 875 A1**
(43) Date of publication of application: **30.03.2016**
(21) Application number: 14186449.6
(22) Date of filing: 25.09.2014
(51) Int. Cl.: C12N 5/0775

(54) **Method for manufacturing of a composition comprising ex vivo expanded human mesenchymal stromal cells**

(71) Applicant: PHILIPPS-UNIVERSITÄT MARBURG, 35037 Marburg (DE); Justus-Liebig-Universität Gießen, 35390 Gießen (DE)
(72) Inventor: Brendel, Cornelia, 35043 Marburg (DE); Nold, Philipp, 75045 Walzbachtal (DE); Hackstein, Holger, 35398 Giessen (DE)
(74) Representative: Stumpf, Peter

(57) **Abstract**

The invention disclosed herein describes a method for manufacturing of a composition comprising human mesenchymal stromal cells (MSC) by ex vivo expansion for therapeutic purposes. The method provides a standardized "off-the-shelf" composition comprising human MSC with reliable efficacy, built on a precisely controlled GMP compliant ex vivo expansion manufacturing process that is biologically safe and preserves the T-cell immunosuppressive human MSC function.

## Description

### Background of the invention

Mesenchymal stromal cells (MSC) are characterized by a high capacity of self-renewal and multipotency. Upon adequate stimuli, they can differentiate into fat, bone or cartilage. Human MSC can be isolated from bone marrow or bone marrow aspirate and do not elicit a rejection reaction by the host after allogenic transplantation. In allogenic application cells from healthy donors according to effective tissue regulations are used, in autologous application cells from the patients themselves are used. Beyond this, they have been shown to accumulate in damaged tissue and are potent modulators of immune responses. Taken together, these properties make them good candidates for innovative therapeutic approaches in inflammatory, autoimmunity and alloimmunity disorders. Numerous clinical trials have been conducted in order to explore the regenerative potential, the tissue preservation properties and the anti-inflammatory capabilities of MSC. Furthermore, they are efficient in the treatment of e.g. multiple sclerosis, Crohn's disease and graft-versus-host disease (GvHD). While some trials for application of MSC in acute or chronic GvHD have yielded promising results, the outcomes of others remained below expectation. These inconsistencies as well as the vision to employ MSC safely for therapeutic purposes have evoked the demand for a standardized medicinal product with reliable efficacy and a precisely controlled GMP compliant manufacturing process. However, applying living primary cells to patients always bears the task to verify that biological capacities have been maintained after ex vivo expansion and to consider a maximum of safety issues.

The present invention aims at providing an "off-the-shelf" composition comprising ex vivo expanded human MSC. Optionally, γ-irradiation of ex vivo expanded human MSC is performed for prevention of possible uncontrolled proliferation in the host while their T-cell immunosuppressive function and viability is retained. The invention shows that high passage numbers, long cryopreservation time and lack of post-thaw equilibration time affects T-cell immunosuppressive functions of MSC adversely. Moreover, the present invention shows that hypoxia significantly increases proliferation at preserved T-cell immunosuppressive capacity, thereby avoiding potential deleterious effects of oxidative stress and mimicking physiological conditions of MSC growth in vivo. Beyond this, the present invention describes that C-X-C motif ligand 12 (CXCL12) mRNA levels are associated with the T-cell immunosuppressive capacity of MSC. In summary, the invention provides a valuable method for manufacturing of a biologically safe and efficient composition comprising ex vivo expanded human MSC that can be used for therapy of inflammatory, autoimmunity and alloimmunity disorders.

### Technical problem

Until now, a method providing a standardized composition comprising human MSC with reliable efficacy, built on a precisely controlled GMP compliant ex vivo expansion manufacturing process, is not available. However, the application of living primary cells such as human MSC to patients always bears the task to verify that biological capacities have been maintained after ex vivo expansion and to consider a maximum of safety issues. Therefore, there is a need for an optimized method for manufacturing of an efficient "off-the-shelf" composition comprising human MSC that is safe and preserves the T-cell immunosuppressive MSC function after ex vivo expansion for therapeutic purposes.

### Solution of the problem

The aforementioned technical problem is solved by manufacturing of human MSC by ex vivo expansion in a closed hollow fiber based bioreactor system according to GMP standards using hypoxic cell culture conditions which enhances the proliferation and thus enabling a low number of passaging steps of the ex vivo expanded human MSC. This new method ensures a manufacturing process under tightly controlled conditions, e.g. continuous lactate monitoring. Optionally, the ex vivo expanded human MSC are γ-irradiated. Moreover, T-cell immunosuppressive function can be determined by measuring CXCL12 expression as a surrogate marker. Moreover, senescence is quantified by beta-galactosidase staining of MSC after 5 days of culture, thus ensuring the immunosuppressive function and avoiding potential deleterious effects of senescent cells.

### Description of the invention

The present invention relates to a method for manufacturing of human mesenchymal stromal cells (MSC) by ex vivo expansion for therapeutic purposes, wherein manufacturing comprises the following steps:
(i) cultivation of human MSC under hypoxic conditions,
(ii) passaging of human MSC up to passage number 1 or up to 4,
(iii) determination of senescence of human MSC.

### Origin of human MSC

Ex vivo expanded human MSC are prepared from isolated spongiform bone marrow fragments or bone marrow aspirate of healthy humans for allogenic applications or of patients for autologous applications.
In a most preferred embodiment, freshly isolated human MSC are used for ex vivo expansion according to the present invention.
In another embodiment, human MSC from cryopreservation are used according to the present invention.

### Cultivation of human MSC under hypoxic cell culture conditions according to the present invention

In a preferred embodiment of the present invention, the cell culture contains 1% or up to 10% of oxygen (O₂). In a most preferred embodiment, the cell culture contains 5% oxygen (O₂).

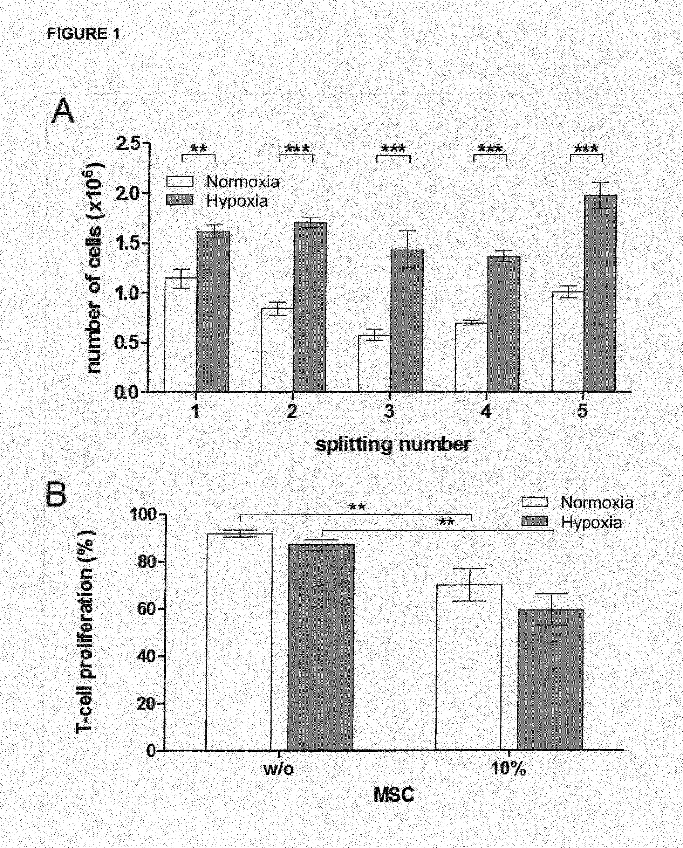

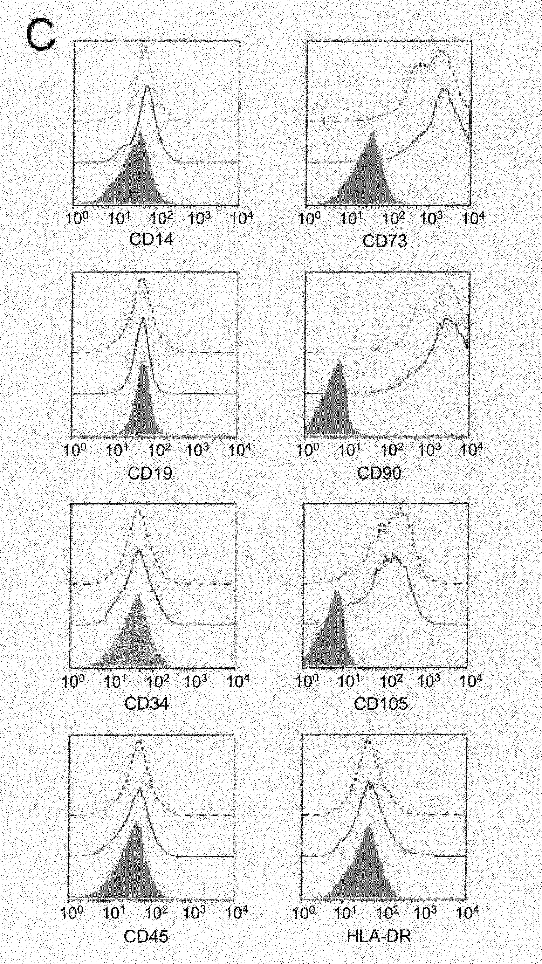

Hypoxic cell culture conditions lead to a 1.4- to 2.5-fold increase of MSC proliferation (Figure 1 A). MSC, either cultivated under normoxic (21% O₂) or hypoxic (1 or up to 10% O₂) conditions, both significantly inhibit T-cell proliferation (Figure 1 B). Of note, hypoxia does not influence T-cell proliferation itself. The presence of characteristic MSC surface markers is shown by flow cytometry as defined by the International Society for Cellular Therapy. Hypoxia-derived MSC prove to have the same phenotype as normoxia-derived MSC. Both express CD73, CD90 and CD105 and lack expression of CD34, CD45, CD19, CD14 and HLA-DR (Figure 1 C).

Hypoxic cell culture conditions according to the present invention therefore markedly increase MSC proliferation without affecting their T-cell immunosuppressive capacity and phenotype, thereby avoiding potential deleterious effects of oxidative stress and mimicking physiological conditions of MSC growth in vivo. Furthermore, hypoxia significantly enhances MSC expansion, allowing for transplantation of MSC with low passage number.

In a preferred embodiment, ex vivo expanded human MSC are passaged up to passage number one (1) or up to four (4).

### Equilibration and passaging of cryopreserved human MSC according to the present invention preserving T-cell immunosuppressive capacity of human MSC

In the present invention the influence of cryopreservation, passaging, and post-thaw cultivation time on MSC T-cell immunosuppressive function is shown.

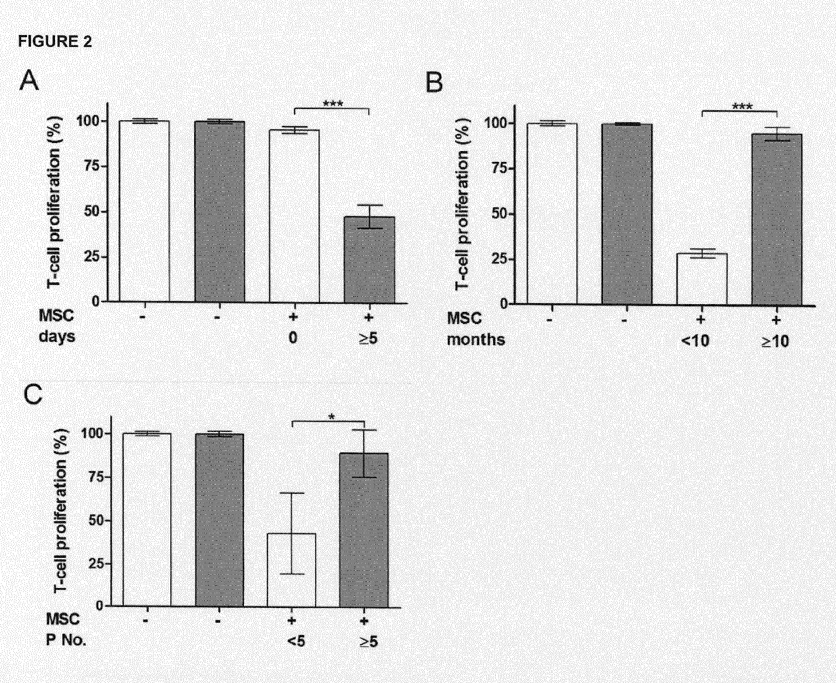

The results presented herein according to the present invention show that freshly thawed MSC do not inhibit T-cell proliferation (Figure 2A), while MSC that are subsequently grown for 5 days or more (median 7 days) significantly reduce T-cell proliferation. Figure 2B shows that cryopreservation for over 10 months (median 11 months) results in poor inhibition of T-cell proliferation compared to storage times under 10 months (median 7 months). MSC with a passage number below 5 (median passage 2) are able to suppress T-cell proliferation to 43%, whereas MSC with a passage number of 5 or more (median 6 passages) lose their T-cell suppressive capacity (Figure 2C). Taken together, a post-thaw equilibration time of up to 5 days is required for restoration of immunosuppressive functions. A high passage number and long cryopreservation time lead to poor T-cell immunosuppressive function of MSC in vitro.

In a preferred embodiment of the present invention, in order to maintain T-cell immunosuppressive function of human MSC from cryopreservation during ex vivo expansion, the following conditions therefore are used:
(1) Use of human MSC from cryopreservation cultures with storage times of less than 10 months,
(2) post-thaw equilibration time of human MSC from cryopreservation cultures of one (1) or up to five (5) days,
(3) passaging of human MSC up to passage number one (1) or up to four (4).

### Optional γ-irradiation of human MSC according to the present invention for inhibition of cell proliferation

According to the present invention, γ-irradiation of ex vivo expanded human MSC is optionally used as a method for prevention of possible uncontrolled proliferation in the host. γ-Irradiation of human MSC abrogates their proliferation while vitality and T-cell immunosuppressive capacity are preserved.

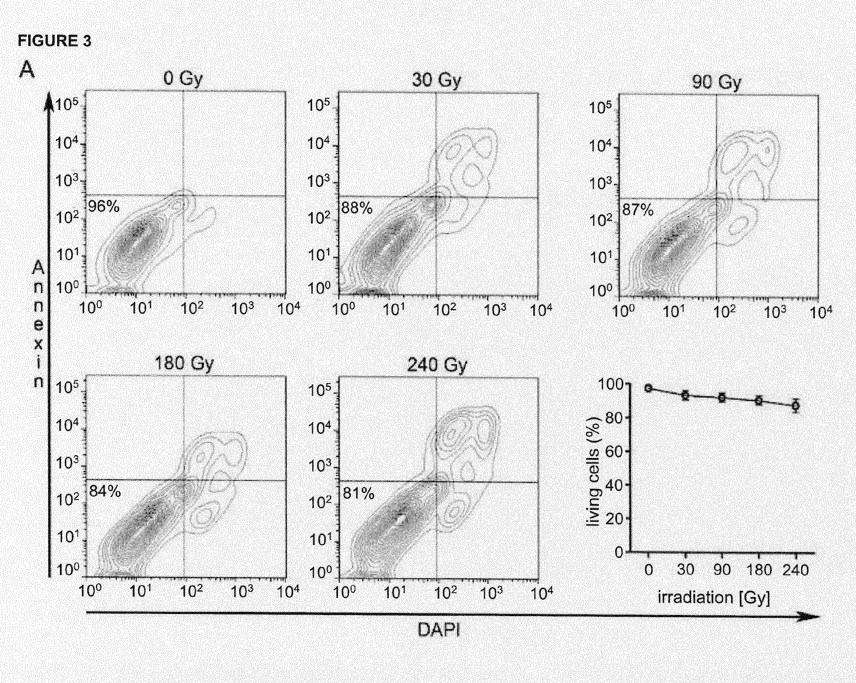

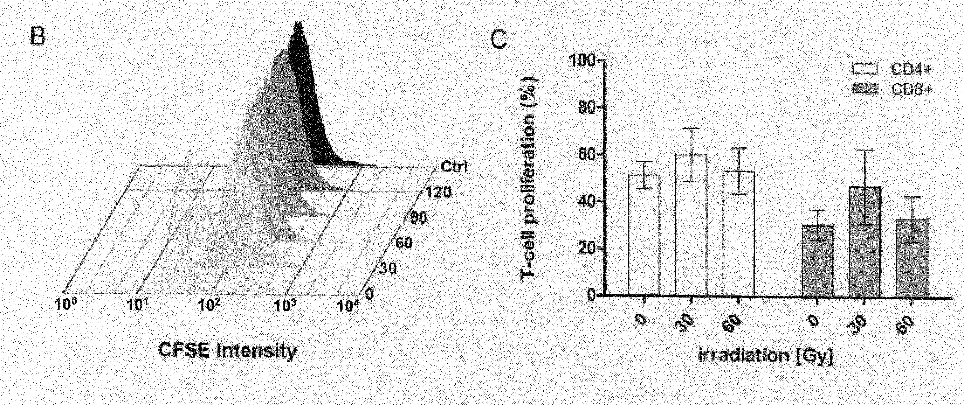

Treatment with γ-irradiation is a common procedure when blood products are administered to patients and alloimmunisation has to be avoided. In the present invention it is described that γ-irradiation also prevents MSC from proliferation. Representative density plots of γ-irradiated MSC show their viability after annexinV/DAPI staining (Figure 3A). Irradiation to 240 Gray (Gy) does not reduce the MSC viability significantly. At 30 Gy, no MSC proliferation is observed anymore which confirms the proliferation-inhibitory effect of γ-irradiation (Figure 3B). Further enhancement of the γ-irradiation dose up to 120 Gy has the same effect. Colchicine-treated MSC serve as control. Furthermore, the effect of γ-irradiation on the immunosuppressive function of MSC by performing a T-cell proliferation assay is determined. Neither irradiation at 30 Gy nor at 60 Gy impair their T-cell immunosuppressive function (Figure 3C).

Statistical analyses reveal no significant differences between the unirradiated control and the two irradiation doses. In summary, therefore 30 Gy are a sufficient dose for a significant reduction of MSC proliferation, while disadvantageous effects on survival and T-cell suppressive function are not observed.

### Determination of senescence and the T-cell immunosuppressive function of ex vivo expanded human MSC according to the present invention.

According to the state of the art it is thought that long-term in vitro culture of human MSC induces senescence which is controlled epigenetically and impairs their physiological function. Therefore, in the present invention the influence of senescence of MSC on T-cell immunosuppressive capabilities of MSC is determined. Senescence of the MSC is determined by β-galactosidase staining. Furthermore, optionally the T-cell immunosuppressive capacity of the MSC at different time points of cultivation is determined by measurement of the mRNA level of the C-X-C motif ligand 12 (CXCL12) using quantitative PCR (qPCR).

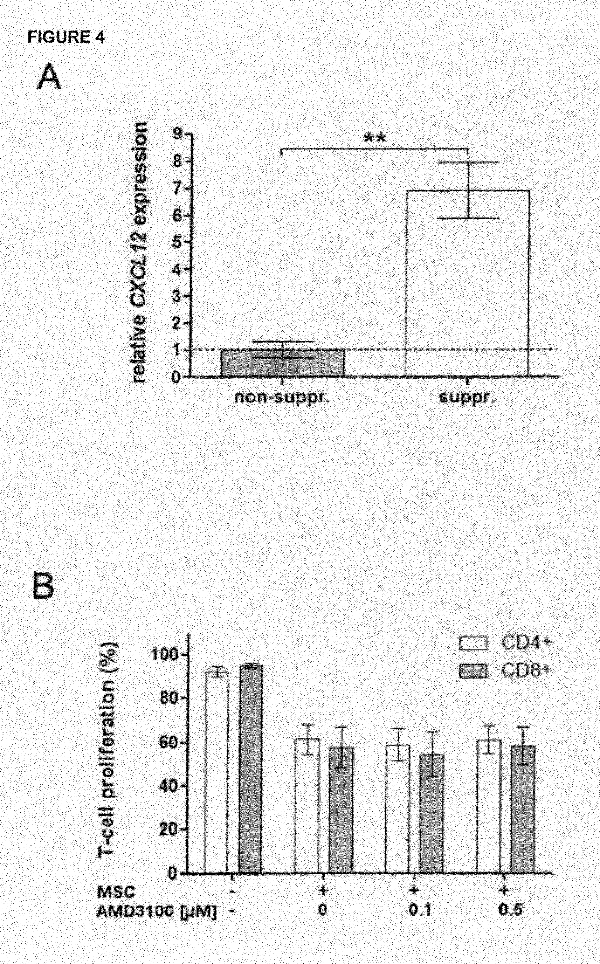

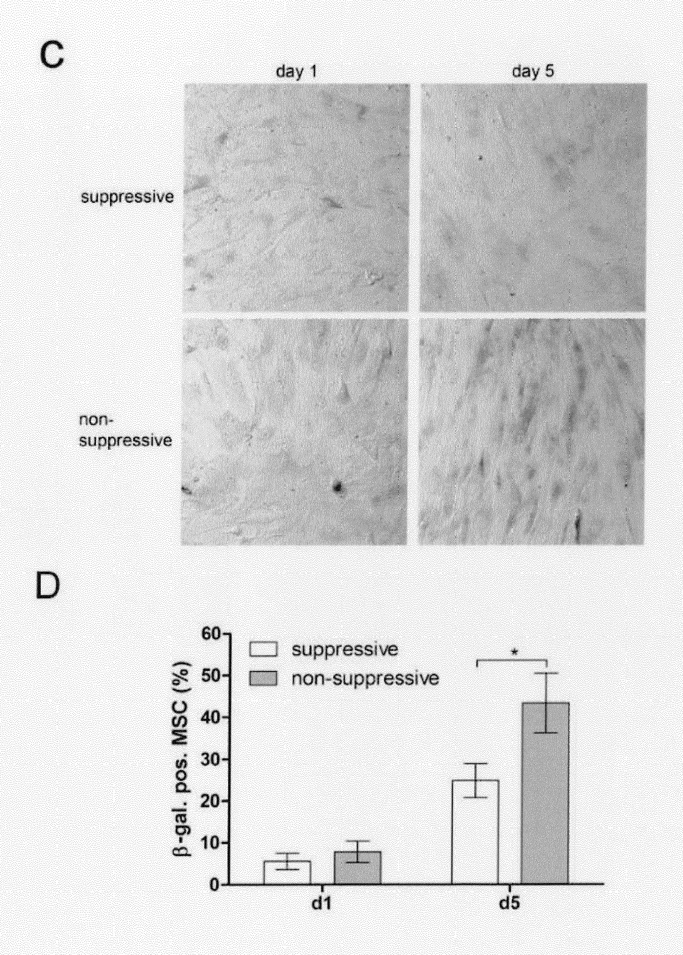

The results show that CXCL12 mRNA expression is 6.9-fold in T-cell immunosuppressive MSC compared to non-immunosuppressive MSC (Fig. 4A). Moreover, the results show that MSC with low T-cell immunosuppressive capacity exhibit a pronounced staining for β-galactosidase 5 days after seeding compared to those MSC that exerted a T-cell immunosuppressive effect (Fig. 4B). A quantification of these results reveals that 43% of the population of non-immunosuppressive MSC are β-galactosidase positive, whereas only 25% of the immunosuppressive MSC are (Fig. 4C).

The data provided herein show that human MSC lacking T-cell immunosuppressive capacity exhibit low expression of CXCL12 and show evidence of senescence according to a pronounced β-galactosidase staining, and MSC with T-cell immunosuppressive function show high CXCL12 expression and less senescence according to a lower β-galactosidase staining than non-immunosuppressive MSC.

In a preferred embodiment of the present invention, β-galactosidase staining is determined in an aliquot of ex vivo expanded human MSC as quality control of human MSC. In a most preferred embodiment, less than 30% of the human MSC in the aliquot exhibit β-galactosidase staining.

In another embodiment, CXCL12 expression is determined in an aliquot of ex vivo expanded human MSC. Preferably, the human MSC of the aliquot exhibit CXCL12 expression at least 2-fold higher compared to non-T-cell-immunosuppressive control cells. The exact quantification is determined for example by probe specific PCR (qPCR; TagMan) or determination of protein levels according to standard procedures.

### Use of ex vivo expanded human MSC for manufacturing of a composition for therapy of inflammatory, autoimmunity, or alloimmunity disorders

Following ex vivo expansion of human MSC according to the present invention the cells are harvested according to standard procedures. An aliquot of the cells is taken to determine senescence of the cells. Those cells that exhibit low senescence (less than 30% β-galactosidase activity of the cells in the aliquot) can be used for manufacturing of a composition for therapy of inflammatory, autoimmunity, or alloimmunity disorders. For manufacturing of the composition, the ex vivo expanded MSC according to the present invention are resuspended in isotonic NaCl solution containing 5% HSA or isotonic NaCl solution containing 5% HSA and 10% DMSO, respectively. The composition can then be used for therapy of inflammatory, autoimmunity, or alloimmunity disorders, for example by intravenous infusion into patients.

### Examples

The examples below illustrate the present invention. However, they shall not be regarded as scope limiting. Shortly, human MSC proliferation and survival after γ-irradiation are determined by 5-carboxyfluorescein diacetate N-succinimidyl ester (CFSE)- and annexinV-DAPI staining, respectively. T-cell proliferation assays are employed to assess the effect of γ-irradiation, passaging, cryopreservation, post-thaw equilibration time and hypoxia on T-cell suppressive capacities of MSC. qPCR and β-galactosidase staining serve as quality assays to determine T-cell immunosuppressive function of human MSC.

### Example 1: Isolation and cultivation of human MSC

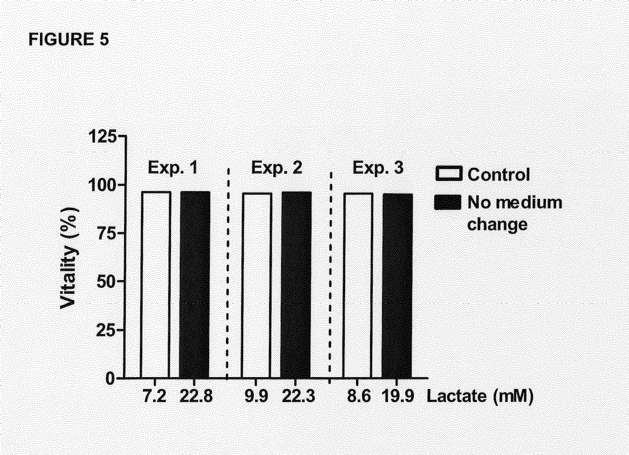

Human MSC are isolated from human spongiform bone marrow fragments or bone marrow aspirate and cultivated for example in a hollow fiber based Quantum bioreactor according to standard procedures (Nold P, Brendel C, Neubauer A, Bein G, Hackstein H, Good manufacturing practice compliant animal-free expansion of human bone marrow derived mesenchymal stromal cells in a closed hollow-fiber-based bioreactor, Biochemical and biophysical research communications, 2013;430(1):325-30). DMEM (Dulbecco's Modified Eagle Medium, Biochrom) is supplemented with 10% good manufacturing practice (GMP)-conform human platelet lysate (HPL) as substitute for fetal bovine serum as described in the state of the art, 1% N(2)-L-alanyl-L-glutamine, and 1% Penicillin/Streptomycin. MSC are cultured at 37°C aid 5% CO₂ in a humidified air chamber under either hypoxic (5% O₂, 5% CO₂, 90% N₂) or normoxic conditions (21% O₂, 5% CO₂, 74% N₂). For passaging human MSC are seeded with approximately 5,000 cells/cm² and culture medium is changed every three days. Alternatively, the culture medium is not changed until the cells are passaged once again according to standard procedures or harvested. Influence of medium change on vitality of human MSC is tested using determination of lactate concentration of the culture medium before passaging or harvesting of cells, respectively, and by flow cytometry of human MSC after DAPI staining (Figure 5). The MSC are harvested after 1 or up to 4 times of passaging according to standard procedures and analyzed according to examples 3 to 7.

### Example 2: Cryopreservation and thawing of human MSC and subsequent cultivation

Human MSC are trypsinized according to standard procedures and centrifuged at 300 x g for 5 min. The pellet is then resuspended in 1.5 ml of freezing medium (containing 10% of DMSO) at a concentration of 10⁶ cells/tube. The cell suspension is pipetted into freezing tubes, which are immediately placed in a Cryo 1° freezing container (Nalgene) and put at -80°C. After 24 h, tubes are transferred to liquid nitrogen. Alternatively, MSC are suspended in isotonic NaCl solution containing 5% human serum albumin (HSA) and 10 % DMSO to a maximum of 10 million cells/ml and frozen in a Biofreeze BV 50 Freezing device (Consarctic) according to standard procedures. Thawing of MSC is conducted as follows: cryogenic storage tubes that were stored for a maximum of 9 months are put in a water bath at 37°C. As soon as possible, the stil semi-frozen solution is transferred to a new vial with at 37°C pre-warmed medium (DMEM, supplemented with 10% GMP-conform human platelet lysate (HPL), 1% N(2)-L-alanyl-L-glutamine, and 1% Penicillin/Streptomycin) in order to dilute DMSO. Cells are immediately washed with phosphate-buffered saline (PBS) and then incubated as described in Example 1 with 5% O₂. The MSC are harvested after 1 or up to 4 times of passaging according to standard procedures and analyzed according to examples 3 to 7.

### Example 3: Human MSC viability, proliferation and T-cell immunosuppressive function after γ-irradiation

Human MSC from example 1 or 2, respectively, are γ-irradiated at different doses via the X-Rad320iX device (Precision X-Ray). MSC viability is analyzed after 7 days with the annexinV apoptosis detection kit (eBiosience). Signal intensities are acquired with a BD LSR II™ device (BD Biosciences) with BD FACS DIVA™. Data are analyzed with FlowJo™ version 9.5.3 (TreeStar Inc.) and GraphPad Prism™ software. Proliferation potential is determined as follows: MSC are stained with 5-carboxyfluorescein diacetate N-succinimidyl ester (CFSE) (Molecular Probes) according to standard procedure before irradiation. The dilution of CFSE by each cell division, reflected by a decrease of signal intensity, serves as an indicator of proliferation. MSC additionally treated with 1mM colchicine (Sigma-Aldrich) serve as a control. Samples are incubated for 7 days. T-cell immunosuppressive function is determined as follows: MSC are subjected to a CSFE-based T-cell proliferation assay according to standard procedures.

### Example 4: Human MSC growth and immunophenotype under hypoxic conditions

In order to analyze the proliferation-promoting effect of hypoxic culture conditions, MSC from example 1 or 2 are seeded with a density of 3,000 cells per cm² and incubated under either normoxic (21 % O₂) or hypoxic (5% O₂) conditions. After 3 days or when cells reach ∼80% of confluence, both normoxia- and hypoxia-derived MSC are harvested using trypsin and counted via hemacytometer. Dead cells are excluded by trypan blue (Sigma-Aldrich) staining. Seeding, cultivation and counting are repeated as before up to splitting number 5. To assess T-cell suppressive capacity, the T-cell proliferation assay is carried out under normoxic and hypoxic conditions as described previously (Nold P, Brendel C, Neubauer A, Bein G, Hackstein H., Good manufacturing practice compliant animal-free expansion of human bone marrow derived mesenchymal stromal cells in a closed hollow-fiber-based bioreactor, Biochemical and biophysical research communications, 2013;430(1):325-30). The immunophenotype of MSC is analyzed after cultivation under normoxic or hypoxic conditions for 7 days. Following surface markers are measured with the human MSC Phenotyping Kit (Miltenyi Biotech) according to the recommendations of the International Society for Cellular Therapy (ISCT) (1): CD14, CD19, CD34, CD45, CD73, CD90 and CD105.

### Example 5: Determination of T-cell immunosuppressive function of human MSC by analysis of CXCL12 expression

CXCL12 expression of human MSC is determined for example by quantitative polymerase chain reaction (qPCR). Therefore, RNA of human MSC is extracted according to standard procedures, e.g. with the RNeasy mini kit (Qiagen). High RNA quality is confirmed with the Experion Bio-Analyzer system (Bio-Rad). 1 µg of RNA is transcribed into cDNA using the QuantiTect SYBR Green PCR kit (Qiagen) according to the manufacturer's instructions. PCR cycling is performed by a CFX96 thermocycler (Bio-Rad). ABL1 or GAPDH are used as housekeeping genes. Primer sequences are: ABL1 sense 5'-tcatgaaagagatcaaacacccta-3', ABL1 antisense 5'-catgaactcagtgatgatatagaacg-3'; CXCL12 sense 5'-gggctcctgggttttgtatt-3', CXCL12 antisense 5'-gtcctgagagtccttttgcg-3'; GAPDH sense 5'-ctcctccacctttgacgctg- 3', GAPDH antisense 5'-tcctcttgtgctcttgctgg-3'. Final primer concentration is 500 nM.

### Example 6: Determination of senescence of human MSC using β-galactosidase staining

β-Galactosidase staining is performed as follows: Human MSC are seeded and incubated for 5 days using standard conditions. Afterwards, β-Galactosidase staining is performed according to standard procedures. Variations from standard procedures are as follows: Fixation time at room temperature is reduced to 3 minutes. During overnight incubation, the cell culture plate is additionally sealed with parafilm (for example Pechiney Plastic Packaging).

### Example 7: Statistics

Data are expressed as mean values ± standard error of the mean. Statistical analyses are performed by either student's t-test or ANOVA test employing the GraphPad Prism^{™} software. Significances: p value of less than 0.05 (*), p less than 0.01 (**), and p less than 0.001 (***).

### Description of the drawings

**Figure 1****.** Effect of hypoxic culture conditions on human MSC proliferation, T-cell suppressive capacity and phenotype. (A) After each splitting, an equal number of human MSC is seeded and incubated under normoxic or hypoxic conditions for up to 3 days or 80% of confluence (Anova test; n = 4, **: p < 0.01, ***: p < 0.001). (B) T-cell proliferation assay after co-culture with human MSC (input 10% of PMBC number) or without (w/o) human MSC under normoxic or hypoxic conditions (right group, Anova test, n ≥ 5, **: p < 0.01). Importantly, oxygen concentration does not affect T-cell proliferation itself (left group). (C) Representative histograms of human MSC-defining surface markers after hypoxic (dashed line) and normoxic (solid line) culture as analyzed by flow cytometry. The solid grey front curve represents the isotype control.
**Figure 2****.** Influence of post-thaw cultivation time, cryopreservation time and passage number on T-cell suppressive capacities of human MSC. (A) T-cell proliferation assay with human MSC which undergo a post-thaw equilibration time of 0 or ≥ 5 days (t-test, n = 6, ***: p < 0.001), (B) with human MSC cryopreserved either for < 10 months or ≥ 10 months (t-test, n = 3, ***: p < 0.001), (C) with human MSC passaged for < 5 times or for ≥ 5 times (t-test, n = 3, *: p < 0.05). In all cases, results are normalized to T-cell proliferation in the absence of the respective human MSC.
**Figure 3****.** Effect of γ-irradiation on survival, proliferation and T-cell suppressive capacity of MSC. (A) Flow cytometric analysis of human MSC survival is determined via annexinV-DAPI staining 3d after irradiation at indicated doses. The diagram represents the results of 3 independent experiments. (B) Representative histogram displaying proliferation of CFSE pre-labeled human MSC, determined by flow cytometric analysis of the human MSC signal 7 days after irradiation at the indicated doses. Colchicine treated MSC serve as non-proliferating control. CFSE intensity is halved by each cell division. (C) T-cell proliferation as measured by CFSE signal intensity after co-culture with human MSC which are previously irradiated at the indicated doses. Differences in T-cell proliferation between irradiated and unirradiated samples are not significant (Anova test, n = 6).
**Figure 4****.** Parameters associated with the T-cell suppressive phenotype of human MSC. (A) CXCL12 expression in T-cell non-suppressive and suppressive human MSC as determined by qPCR (t-test, n = 3, **: p < 0.01). ABL1 expression serves as reference. (B) T-cell proliferation assay with 0, 0.1, or 0.5 µM AMD3100. Differences between AMD3100-treated and untreated samples are not significant (Anova test, n = 9). (C) Representative light microscope images of suppressive (top) and non-suppressive human MSC (bottom) stained for β-galactosidase at indicated time points after seeding. Magnification 80x. (D) Quantification of (C), indicating the percentage of β-galactosidase positive cells (t-test, n = 4, *: p < 0.05).
**Figure 5****.** MSC vitality in relation to the lactate concentration of the medium. Medium of control cells (white bars) is changed every three days for regular nutrient addition and removal of metabolism products, while others (black bars) are incubated for 7 days without medium change. Vitality is measured after DAPI staining by flow cytometry. Experiments are conducted with different MSC batches.

The following sequences referred to herein are shown in the accompanying sequence listing:
**SEQ ID No. 1:** ABL1 sense 5'-tcatgaaagagatcaaacacccta-3'
**SEQ ID No. 2:** ABL1 antisense 5'-catgaactcagtgatgatatagaacg-3'
**SEQ ID No. 3:** CXCL12 sense 5'-gggctcctgggttttgtatt-3'
**SEQ ID No. 4:** CXCL12 antisense 5'-gtcctgagagtccttttgcg-3'
**SEQ ID No. 5:** GAPDH sense 5'-ctcctccacctttgacgctg- 3'
**SEQ ID No. 6:** GAPDH antisense 5'-tcctcttgtgctcttgctgg-3'

### Abbreviations

- CFSE: 5-carboxyfluorescein diacetate N-succinimidyl ester
- CXCL12: (C-X-C motif) ligand
- GMP: Good manufacturing practice
- GvHD: Graft-versus-host disease
- Gy: Gray
- IDO: Indoleamine 2,3-dioxygenase
- MSC: Mesenchymal stromal cell
- qPCR: Quantitative polymerase chain reaction
- DAPI: 4',6-diamidino-2-phenylindole
- DMEM: Dulbecco's modified Eagle medium
- HPL: Human platelate lysate
- HSA: Human serum albumin
- ABL1: Abelson murine leukemia viral oncogene homolog 1
- GAPDH: Glyceral aldehyde phosphate dehydrogenase
- AMD3100: also known under brand name Plerixafor
- HLA-DR: Human leucocyte antigen DR

## Claims

1. Method for manufacturing of a composition comprising human mesenchymal stromal cells (MSC) by ex vivo expansion, wherein manufacturing comprises the following steps:
(i) cultivation of human MSC under hypoxic conditions,
(ii) passaging of human MSC up to passage number 1 or up to 4,
(iii) determination of senescence of human MSC.

2. Method according to claim 1, wherein the human MSC are isolated from spongiform bone marrow fragments or bone marrow aspirate of healthy humans for allogenic applications or of patients for autologous applications.

3. Method according to claim 2, wherein the human MSC are freshly isolated from spongiform bone marrow fragments or bone marrow aspirates of healthy humans or patients.

4. Method according to claim 2, wherein the human MSC are cryopreserved human MSC stored for up to nine (9) months and are equilibrated after thawing for one (1) or up to five (5) days.

5. Method according to claims 1 to 4, wherein the human MSC are cultivated under hypoxic conditions with one (1) % or up to ten (10) % O₂.

6. Method according to claim 5, wherein the human MSC are cultivated under hypoxic conditions with five (5) % O₂.

7. Method according to claims 1 to 6, wherein the human MSC are γ-irradiated following step (ii) with one (1) or up to twohundredforty (240) Gray (Gy).

8. Method according to claim 7, wherein the human MSC are γ-irradiated with thirty (30) Gray (Gy).

9. Method according to claims 1 to 8, wherein senescence of ex vivo expanded human MSC is determined in an aliquot of human MSC of step (ii) by β-galactosidase staining.

10. Method according to claim 9, wherein the β-galactosidase staining is performed after five (5) days of cultivation of the human MSC.

11. Method according to claims 9 to 10, wherein less than 30% of the human MSC in the aliquot exhibit β-galactosidase staining.

12. Method according to claims 1 to 8, wherein T-cell immunosuppressive function of ex vivo expanded human MSC is determined by analysis of CXCL12 expression in an aliquot of human MSC of step (ii).

13. Method according to claim 12, wherein the human MSC of the aliquot exhibit CXCL12 expression at least 2-fold higher compared to non-T-cell-immunosuppressive control cells.

14. Use of composition manufactured according to claims 1 to 13 for therapy of inflammatory, autoimmunity, or alloimmunity disorders.


SEQUENCE LISTING

<110>  Philipps-Universität Marburg

<120>  Method for manufacturing of a composition comprising ex vivo
       expanded human mesenchymal stromal cells

<130>  TM 804

<160>  6

<170>  PatentIn version 3.5

<210>  1
<211>  24
<212>  DNA
<213>  Homo sapiens


<220>
<221>  misc_feature
<222>  (1)..(24)

<220>
<221>  misc_feature
<222>  (1)..(24)
<223>  Sense primer

<400>  1
tcatgaaaga gatcaaacac ccta                                              24


<210>  2
<211>  26
<212>  DNA
<213>  Homo sapiens


<220>
<221>  misc_feature
<222>  (1)..(26)

<220>
<221>  misc_feature
<222>  (1)..(26)
<223>  Antisense primer

<400>  2
catgaactca gtgatgatat agaacg                                            26


<210>  3
<211>  20
<212>  DNA
<213>  Homo sapiens


<220>
<221>  misc_feature
<222>  (1)..(20)

<220>
<221>  misc_feature

<222>  (1)..(20)
<223>  Sense primer

<400>  3
gggctcctgg gttttgtatt                                                    20


<210>  4
<211>  20
<212>  DNA
<213>  Homo sapiens


<220>
<221>  misc_feature
<222>  (1)..(20)

<220>
<221>  misc_feature
<222>  (1)..(20)
<223>  Antisense primer

<400>  4
gtcctgagag tccttttgcg                                                    20


<210>  5
<211>  20
<212>  DNA
<213>  Homo sapiens


<220>
<221>  misc_feature
<222>  (1)..(20)

<220>
<221>  misc_feature
<222>  (1)..(20)
<223>  Sense primer

<400>  5
ctcctccacc tttgacgctg                                                    20


<210>  6
<211>  20
<212>  DNA
<213>  Homo sapiens


<220>
<221>  misc_feature
<222>  (1)..(20)

<220>
<221>  misc_feature
<222>  (1)..(20)
<223>  Antisense primer

<400>  6
tcctcttgtg ctcttgctgg                                                    20